# EUROPEAN PATENT SPECIFICATION

(11) **EP 0 288 325 B1**
(45) Date of publication and mention of the grant of the patent: **22.06.1994**
(21) Application number: 88303717.8
(22) Date of filing: 25.04.1988
(51) Int. Cl.: C12N 15/00, C12N 9/00, C12N 1/14, C12N 1/20, C12P 37/00

(54) **DNA Encoding isopenicillin N-synthetase from A.Nidulans, its use and vectors encoding it.**
Für A.Nidulans-Isopenicillin-N-Synthetase codierende DNS, deren Verwendung und dafür codierende Vektoren
ADN encodant la synthétase N d'isopénicilline de A.nidulans, son utilisation et vecteurs encodant celle-ci

(30) Priority: 24.04.1987 GB 8709708
(43) Date of publication of application: 26.10.1988
(73) Proprietor: Antibioticos S.A., 28015 Madrid (ES)
(72) Inventor: Soto, Miguel Angel Penalba, 28008-Madrid (ES); Vidal, Daniel Ramon, 28006-Madrid (ES); Ortega, Agustin Perez-Aranda, 28035-Madrid (ES)
(74) Representative: Ablewhite, Alan James

(56) References cited:
- EP-A- 0 200 425
- EP-A- 0 225 128
- MOL. GEN. GENETICS, vol. 202, 1986, pages 271-275, Springer-Verlag; D.J. BALLANCE et al.: "Gene cloning in Aspergillus nidulans: isolation of the isocitrate lyase gene (acuD)"
- JOURNAL OF GENERAL MICROBIOLOGY, vol. 122, 1981, pages 339-343, SGM, GB; J.F. MAKINS et al.: "Intergeneric cosynthesis of penicillin by strains of penicillium chrysogenum, P. chrysogenum/notatum and Aspergillus nidulans"
- GENE, vol. 57, no. 2-3, 1987, pages 171-181, Elsevier Scinece Publishers B.V., Amsterdam, NL; D. RAMON et al.: "Cloning and characterization of the isopenicillin N synthetase mediating the formation of the beta-lactam ring in Aspergillus nidulans"
- JOURNAL OF GENERAL MICROBIOLOGY, vol. 129, part 9, September 1983, pages 3027-3033, Crown Copyright, GB; J.F. MAKINS et al.: "The genetic location of three mutations impairing pencillin production in Aspergillus nidulans"
- Proc. Royal Society London B (1990), vol. 241, pp. 164-169, M. Penalva et al.

## Description

### SUMMARY

The present invention is related to the discovery of a novel DNA compound isolated from the genome of the filamentous fungus Aspergillus nidulans. This DNA compund has been cloned in E. coli expression vectors and the protein product encoded by this DNA compound has been identified and shown to have activity of isopenicillin N synthetase, a key enzyme of the biosynthetic pathway of β-lactam antibiotics. A. nidulans is known to produce penicillins but so far no DNA compounds encoding any of the activities of the pathway had been isolated. Moreover, no activity of isopenicillin N-synthetase had been found in extracts of A. nidulans cells, so novelty of the invention is clear. The novel DNA compound encoding isopenicillin N synthetase has been completely sequenced and the amino acid sequence of the protein product deduced. This DNA compound, as well as recombinant DNA vectors containing it, are the object of the present invention.

It is now well established that the biosynthesis of penicillins and cephalosporins occurs in Penicillium chrysogenum, Cephalosporium acremonium and some species of the genus Streptomyces via formation of a tripeptide (L-α-aminoadipyl)-L-cysteinyl-D-valine, from here after abbreviated as LLD-ACV. This tripeptide is converted to the first β-lactam compound of the pathway, whose name is isopenicillin N, by an enzyme which requires Fe⁺⁺, ascorbate, and oxygen and that has been named isopenicillin N-synthetase, and that from here after will be named IPNS. This conversion has been shown to occur in cell-free extracts from P. chrysogenum, C. acremonium and S. clavuligerus.

The genes cooling for the C. acremonium (Samson et al., 1985, Nature, 318, 191-194) and P. chrysogenum (Corr et al., 1986, Gene, 48, 257-266) IPNS enzymes have been cloned and sequence.

Although the imperfect fungus P. chrysogenum is used on industrial scale production, other moulds are capable of synthesizing penicillin. One of these moulds is the ascomycete Aspergillus nidulans. This fungus presents several advantages over P. chrysogenum at culture conditions and manipulation level. For instance, it grows at a much higher rate than P. chrysogenum in a wide range of temperatures from 28°C to 37°C. An Other important advantage that will be recognized by those skilled in the art of genetic improvement of strains for overproduction of microbial products is that A. nidulans has a sexual cycle that can be used for genetic improvement as well as a very efficient parasexual cycle. Genetic recombination can thus easily be achieved. However, A. nidulans is not used for penicillin production due to the very low penicillin titres that synthesizes, even in overproducing strains, that are several orders of magnitude under the titres produced by P. chrysogenum.

On the other hand, A. nidulans is one of the best-known eukaryotic organisms and has become a model system for studying mechanisms of control of gene expression in eukaryotes, mainly due to the possibility of sophisticated genetic analysis, as well as to the metabolic versatility of this organism. Furthermore, many genes of this organism have been cloned, and those skilled in the art of fermentation technology will easily recognize how some of them would notably decrease the cost of the fermentation process by using cheaper substrates when de-regulated in the proper way by recombinant DNA techniques.

Techniques for manipulation of A. nidulans by recombinant DNA procedures are also available. Very efficient transformation systems have been described. Transformation occurs by integration in the host genome and can be manipulated in such a way that the transforming DNA is directed to specific regions of the genome, allowing thus precise gene replacements. Furthermore, strong regulated promoters have been described, as well as strong transcriptional termination signals. These regulatory signals could be used to overproduce certain gene products like those of the β-lactam biosynthetic pathway, that could lead to a dramatic increase in penicillin titres. On the other hand, it is well known that integration of transforming DNA can occur in tandem and that these types of integration events can easily be identified by standard techniques. Thus, increasing the copy-number of a particular gene or genes, like those of the β-lactam pathway, is relatively easy to achieve. Moreover, integration of the transforming DNA into the genome ensures a very high mitotic stability, a characteristic that is very important when producing microbial metabolites at industrial scale fermentation.

The DNA compound of the present invention encodes a protein product which has one of the activities of the β-lactam biosynthetic pathway. Taken into account the above mentioned consideranda, it can be introduced in one of the many available A. nidulans vectors and its expression can be increased in some of the ways above described. Thus, it can be used to manipulate A. nidulans by recombinant DNA techniques in such a way that penicillin titres produced by this mould would be interesting from an industrial point of view. In combination with mutations already described in A. nidulans or with other recombinant DNA manipulations it can be used to convert A. nidulans into a microorganism suitable for industrial production of penicillins.

A second aspect that has to be taken in mind about the DNA compound of the present invention is that it can be overexpressed in E. coli up to a level that the protein product that it encodes constitutes the major protein synthesized in the genetically engineered bacteria. Not only IPNS does accept the natural substrate LLD-ACV, other tripeptides can be accepted (Antibióticos, S.A., European Patent Appplication 86305447.4) and availability of pure protein product in substantial amounts can be easily achieved. Determination of the three-dimensional structure of the active site is thus feasible and could allow us to modify the active site by mutagenesis in such way that the efficiency of the reaction with a particular substrate is increased several-fold. "In vitro" reactions could then lead to enzymatic synthesis of new β-lactam antibiotics, or, once the gene is modified, to the synthesis of new antibiotics by fermentation by reintroducing the modified DNA compound again in a suitable A. nidulans host.

The following sections will describe particular aspects of the present invention.

### Detailed description of the invention

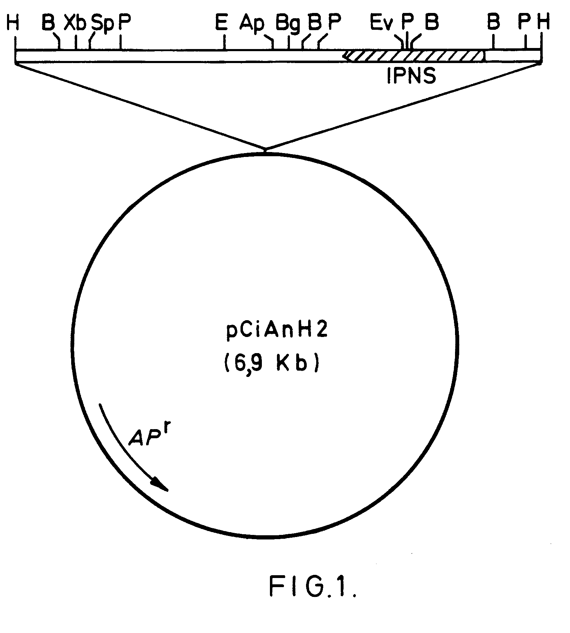

The present invention comprises DNA compounds and recombinant DNA cloning and expression vectors which encode the novel isopenicillin N synthetase gene, a DNA compound so far undescribed in Aspergillus nidulans. The sequence of this novel DNA compound is shown in Annex 1. Only the coding strand of the double-stranded DNA molecule is shown for clarifying in the 5′ -- 3′ orientation. The first nucleotide corresponds to the A of the ATG triplet that encodes the initiator methionine. The last nucleotide corresponds to the third nucleotide of the stop codon TAG that is used in the DNA compound of the present invention. In fig.1, A means deoxyadenyl, G is deoxyguanyl, C is deoxycytidyl and T is thymidyl.

The novel DNA compound whose structure is described in detail in Appendix 1 encodes a polypeptide whose amino acid sequence is depicted in Appendix 2. Those skilled in the art will recognize that this novel DNA compound, isolated from a natural microorganism can be synthesized totally using automated DNA synthesizers, such as the Applied Biosystems, Inc. Model 380A. We are also conscious that due to the degenerate nature of the genetic code, the protein encoded by the novel DNA compound could be encoded by alternative DNA sequences. These are included in the present invention.

Besides, any natural genetic variant arising from the novel DNA compound described in the present invention is considered to be equivalent of it. The novel IPNS activity encoding DNA compound was isolated from a genomic bank constructed in bacteriophage lambda EMBL4 of Aspergillus nidulans strain bioA1, that can be obtained from Culture Collections. Once the recombinant phages which contained the gene were identified, a 4.2 Kb HindIII DNA fragment containing the compound of the present invention was cloned in the HindIII site of plasmid pUC13 to yield pCiAnH2. E. coli DH1 containing recombinant plasmid pCiAnH2 has been deposited under the Budapest Treaty in The National Collection of Industrial and Marine Bacteria, address: Torry Research Station, P.O. Box 31, 135 Abbey Road, Aberdeen, AB9 8DG, Scotland; under accesion number: NC1B 12450. The gene was contained in two adjacent BamHI fragments that were completely sequenced by the well known Sanger's method (Sanger, F., Nickens, S. and Coulson, A.R. (1977) Proc. Natl. Acad. Sci. USA 78, 3128). The DNA compound of the present invention encodes a polypeptide, as deduced from the nucleotide sequence with a molecular mass of 37.480 g.mol⁻¹. The novel DNA sequence was then cloned in an E. coli expression vector pIMIII-A1 to yield plasmid pIM-Cic. When the construction is transformed in the appropriate E. coli cells, the protein encoded by the DNA compound is the major polypeptide found in lysate of these E. coli cells (see Examples). Further, IPNS activity can be easily detected in these E. coli cells (see Examples). Plasmid pCiAnH2 is interesting because it contains single restriction enzyme sites which can be used to introduce appropriate A. nidulans transformation markers and the resulting chuimaeric molecules used to transform suitable A. nidulans strains. It has not escaped to our notice that the mentioned marker could be one that easily allows detection of multicopy tamdem transformants, for instance, the amdS gene.

### Example 1

### Construction of pCiAnH2.

A 4.2 Kb DNA fragment containing the novel DNA compound (i.e., the gene coding for the isopenicillin N synthetase from A. nidulans) was isolated by digestion with HindIII of the DNA of a lambda EMBL4 vector which contained an insert of ≈ 15 Kb of A. nidulans DNA with HindIII. The mentioned fragment was separated from other DNA fragments by gel electrophoresis in a 1% agarose gel prepared in 40 mM Tris-acetate, 2 mM EDTA, pH 8.0. The desired DNA fragment was isolated from the gel by electroeluction. The 4.2 Kb DNA fragment (100 ng) was mixed with 100 ng of plasmid pUC13 which had previously been digested with HindIII and incubated in a final volume of 10 »l in a buffer that contained 50 mM Tris-HCl pH 7.4, 10 mM Cl₂Mg, 10 mM DTT, 1 mM ATP, and 100 »g/ml of bovine serum albumin in the presence of 0.3 units of DNA ligase (New England Biolabs, Inc.) for 15 hours at 15^{o}C. All restriction enzyme digestions were made in the conditions recommended by the manufacturer.

A culture of E. coli strain DH5 (Bethesda Research Laboratories, Inc.) was grown at 37^{o}C with shaking to O.D⁵⁵⁰ = 0.3. in broth: 2% (w/v) Bacto Tryptone Difco, 0.5% (w/v) Bacto Yeast Extract Difco, 0.4% (w/v) MgSO₄ and 10 mM KCl adjusted to pH 7.6 with KOH. This culture was diluted 20 times with prewarmed broth and grown at 37^{o}C with shaking to O.D⁵⁵⁰ = 0.48. Cells were chilled in ice and spun at 5000 rpm for 5 minutes at 4^{o}C in the GSA rotor (Sorvall) 100 ml of starting culture were resuspended in 30 ml of ice-cold TfBI: 100 mM RbCl₂ 50 mM MnCl₂, 30 mM potassium acetate, 10 mM CaCl₂, 15% (v/v) glycerol adjusted to pH 5.8 with 0.2 M acetic acid.

After resuspension, the cells were spun down again in the same conditions and resuspended in 4 ml (per 100 ml of starting culture) of ice-cold TfBII: 10 mM MOPS (Sigma) pH 7.0, 10 mM RbCl₂, 75 mM CaCl₂, 15% (v/v) glycerol and dispensed in 0.2 ml aliquots into 3 ml sterile plastic tubes which were stored at -80^{o}C until use.

For transformation cells were thawed at room temperature until just liquid and put on ice for 5 minutes. Half of the ligation mixture (5 »l) mentioned in the first paragraph of this example was added to the cells which were left on ice for 20 minutes and then incubated for 2 minutes at 37^{o}C and 1 minute further at 0^{o}C. The transformed cells were then transferred to a 10 ml tube containing 1 ml of broth which was gently shaken for 60 minutes at 37^{o}C. Appropriate dilutions were made and plated on L-agar plus 50 »g/ml of ampicillin. Plates were incubated O/N at 37^{o}C.

Twenty isolated ampicillin resistant colonies were streaked in fresh L-medium containing ampicillin and the size of the plasmid from these colonies was analyzed by a colony-lysis procedure using appropriate molecular weight standards. One clone containing a plasmid with the expected 4.2 Kb insert was chosen for large-scale purification. For that purpose, 0.5 ml of L-broth containing 150 »g/ml of ampicillin were inoculated with a couple of colonies from the streak of the chosen clone. The culture was grown overnight at 37^{o}C. Cells were pelleted by centrifugation at 6000 rpm for 10 minutes at 4^{o}C in the Sorvall GSA rotor and resuspended in 40 ml of 50 mM glucose, 25 mM Tris-HCl pH 8.0, 10 mM EDTA, 5 mg/ml lysozyme and incubated for 10 minutes at room temperature. After this time, 80 ml of 0.2 M NaOH, 1% SDS (w/v) were added, mixed by swirling and kept in ice for 5 minutes. Then 40 ml of 5 M potassium acetate pH 4.8 were added and the mixture was kept in ice for 1 hour. The precipitate was sedimented in the Sorval GSA rotor at 4°C and the supernatant containing the plasmid DNA was mixed with 0.6 V of isopropanol to precipitate nucleic acids. The precipitate was collected at 8000 rpm for 5 minutes in the GSA rotor at room temperature. The pellet was drained and resuspended in 7 ml of 10 mM Tris-HCl, 1 mM EDTA. The solution was neutralized with 2 M Tris-base and made up the volume to 8 ml with TE buffer. Then 8 g of cesium chloride were dissolved and when this was achieved, the solution was mixed with 1 ml of an ethidium bromide solution (10 mg/ml in water). The solution was centrifuged for 48 hours at 45000 rpm and 20^{o}C in a Beckman Ti65 rotor. The band containing the covalently closed circular plasmid DNA was identified under long wave U.V. light and collected by side puncture of the tube using a 21 gauge needle. Ethidium bromide was extracted several times with an equal volume of ispropanol saturated with cesium chloride nd the DNA solution was dialyzed against several changes of TE at 4^{o}C.

This purified plasmid was used to construct a restriction map by classical techniques and the result of such a map is represented in fig. 1. Dashed area in the figure indicates the position and direction of transcription of the novel DNA compound, i.e: the A. nidulans IPNS genes structural characterization of the DNA compound.

This characterization was achieved by DNA sequencing. The genomic region comprising the IPNS gene was sequenced by the method of Sanger using subcloning in M13 mp8 or M13 mp9 for obtaining single-stranded templates necessary for the sequencing reaction. All the restriction sites used for subcloning were sequenced through.

### Expression of A. nidulans IPNS gene in E. coli. Preparation of plasmid pIM-Cic.

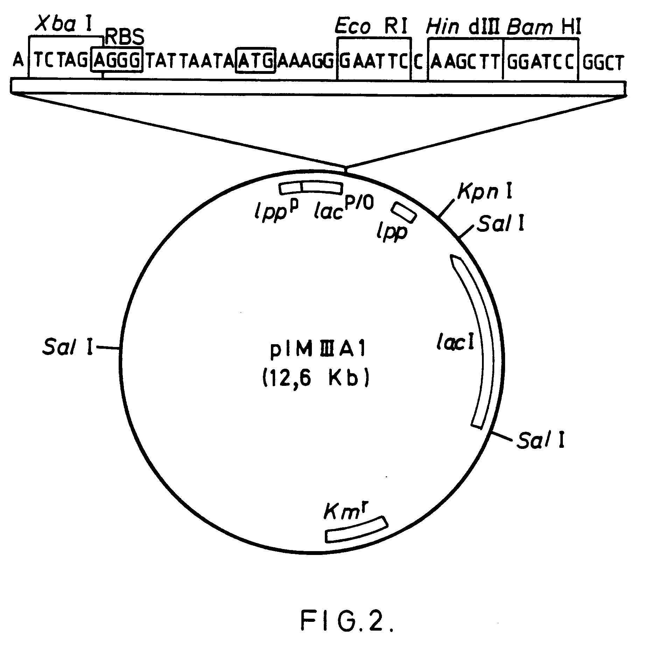

We have used expression plasmid pIM-IIIA1 (fig. 2) for overexpression of the A. nidulans IPNS gene in E. coli.

This plasmid, (Masui, Y., Coleman, J. and Inouye, M. Multipurpose expression cloning vehicles in E. coli. In experimental Manipulation of gene expression. Ed. M. Inouye. Academic Press N.Y., 1983, pp. 15-32), 12.6 Kb long, encodes a kanamicin-resistance gene and contains a ts mutation that affects its copy number in such a way that the number of molecules of plasmid per cell is increased from about 15 at 30^{o}C up to 2000 molecules per cell at the restrictive temperature (37^{o}C). The cloning region of PIM-IIIA1 (see fig. 2) contains the strong E. coli promoter of the lipoprotein gene followed by the promoter-operator region of the lactose operon. Thus, transcription can be controlled by the lactose repressor. Downstream of these transcriptional control units is the ATG corresponding to the initiator methionine of the lipoprotein followed by the first nucleotides of the structural gene. Fourteen base pairs before the ATG there is a single XbaI site and five nucleotides downstream the ATG a synthetic DNA sequence which contains targets for BamHI, EcoRI and HindIII that are unique throughout the plasmid. This four single restriction targets can be used to clone the foreign DNA in this vector. The vector also includes the gene that codifies for the lactose repressor and after the partial sequence of the lipoprotein structural gene, the transcriptional terminator of the mentioned gene.

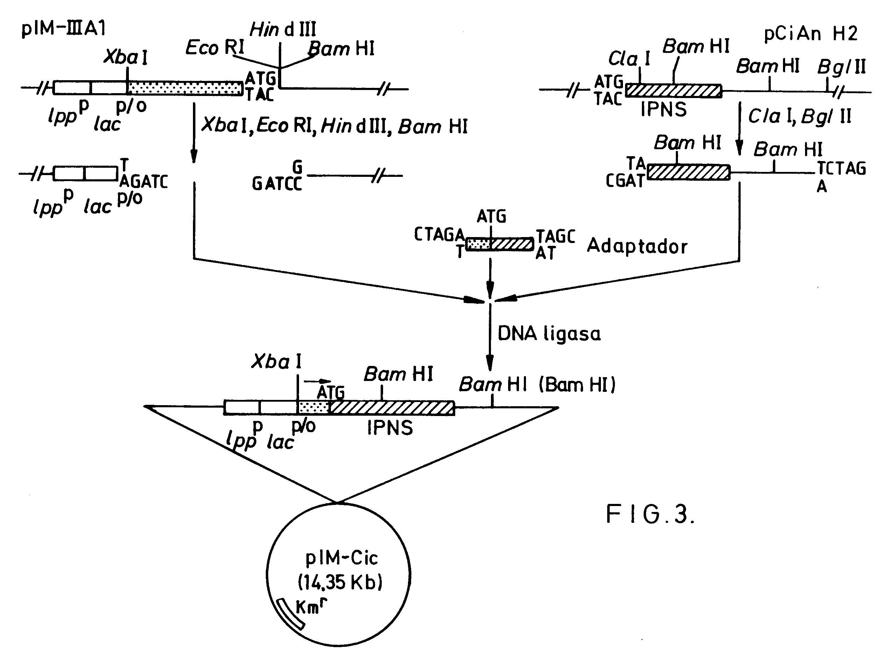

Fig. 3 summarizes construction of pIM-Cic. Digestion of pCiANH2 (example 1, fig. 1) with ClaI and BglII yields a 1.7 Kb fragment containing all but the 33 first nucletoides of the A. nidulans IPNS coding region and around 700 bp downstream the translational terminator. Thus, plasmid pCiANH2 was digested with ClaI and BglII and the resulting DNA fragments were electrophoresed through a 3.5% poliacrilamide gel in 90 mM Tris-borate, 2 mM EDTA pH 8.0. The fragments were stained with methylene blue and the 1.7 Kb ClaI-BglII was identified, sliced from the gel and eluted overnight in a buffer containing 0.5 M ammonium acetate, 1 mM EDTA. The solution containing the fragment was filtered through glass-wool and extracted twice with phenol saturated with 10 mM Tris-HCl, 1 mM EDTA ph 8.0. The residual phenol was extracted with ether and the residual ether eliminated under a nitrogen stream. The fragment was finally ethanol precipitated, and taken up in a small volume of Tris-HCl 10 mM pH 8.0, 1 mM EDTA and stored until use.

Two oligonucleotides were then synthesized in an Applied Biosystems, Inc. 380A DNA synthesizer. The oligonucleotides were complementary and after purification in the conditions described by the manufacturer of the DNA Synthesizer were hybridized in 0.1xSSG at 65^{o}C overnight. After reannealing, they formed an adapter which reconstructed the precise sequence between the XbaI site of pIM-IIIA1 and the ATG corresponding to the lipoprotein coding region. This ATG was followed by the 33 first nucleotides of the ORF present between the initiation codon of IPNS gene and the ClaI site. Both oligonucleotides were designed in such a way that they left overhanging ClaI and XbaI cohesive ends.

Finally, plasmid pIM-IIIA1 was digested with XbaI and BamHI (compatible overhanging ends with BglII) and with HindIII and EcoRI (to prevent self-ligation of the vector). The three fragments were mixed in a 1:1:1 molar proportion and the mixture used to transform E. coli DH1 as described in example 1. An scheme of the construction is shown in fig. 3. Most of the transformants contained the expected recombinant plasmid, that was named pIM-Cic (see fig. 3). One of the clones chosen for further experiments was purified as described in example 1 (except that the selective pressure was maintained for kanamicin-resistance during growth) and was sequenced from the XbaI site to verify that the construction was correct at the nucleotide level.

### Example 3

### Detection of a polypeptide with the molecular mass of IPNS by polyacrilamide gel electrophoresis.

Plasmid pIM-Cic was transformed into E. coli W3110 (lacI^{q}). A culture of this strain containing pIM-Cic was grown in L-broth containing kanamycin (12.5 »l/ml) at 30^{o}C with shaking up to an O.D⁶⁰⁰ = 0.6. The culture was then distributed in two samples and IPTG was added to one of them (final concentration 2 mM). Both cultures were then incubated at 37^{o}C for three hours. 1 ml samples were taken from both cultures, the cells were collected by centrifugation in a Beckman microfuge and resuspended in 100 ul of sample buffer (Tris-HCl 0.0625M, pH 6.8, 2% SDS (w/v), 5% β-mercaptoethanol 5% (v/v) 6 M urea and 0.05 mg/ml fo bromophenol blue). Samples were boiled for 3 minutes and 10 »l samples were loaded on a 10% polyacrilamide gel using the discontinous system of Laemmli (Laemmli, U.K. (1970. Nature. London 277, 680). The gel was run at 30 V overnight plus 2 additional hours at 150 V and stained with Coomassie blue. A polypeptide with a molecular mass of 40 Kd was present in E. coli W3110 cells transformed with pIM-Cic and induced with IPTG and temperature, and absent in control cells transformed with pIM-IIIA1 or in untransformed cells. The protein was synthesized at low levels in cells not induced with IPTG, showing that this polypeptide is under the control of the lac operator region from pIM-Cic and this should correspond to the IPNS gene product. Discrepancy between the observed molecular mass in an SDS-polyacrilamide gels and the real molecular weight has been observed for many proteins.

### Example 4

### IPNS activity is detectable in E. coli cells transformed with pIM-Cic and induced with IPTG and termperature.

W3110 cells, transformed either with pIM-IIIA1 or with pIM-Cic, were induced with IPTG and temperatures as described in example 3. 3 hours after induction, 1-ml samples were centrifuged in the microfuge at 4^{o}C and each pellet was resuspended in 0.5 ml of 50 mM Tris-HCl pH 8.0, 10 mM KCl, 10 mM MgSO₄ and lysed by sonication. Cell debries were pelleted by centrifugation at 20.000xg for 20 minutes at 4^{o}C.

The supernatants were used immediately in the cyclation assay. In a final volume o 100 »l, the reaction mixture contained 10 »l of 2 mM LLD-ACV, 4 »l of 0.1 M dithiothreitol, 2.5 »l of FeSO₄ 4 mM, 2.5 »l of ascorbic acid, 75 »l of cell-free extract and H₂O to complete the final volume. Controls were made with no extract or with no substrate. Reactions were carried out for 30 minutes at 25^{o}C and stoped by addition of an equal volume of methanol. 100 »l samples were placed in wells of 7 mm diameter made in plates of Tryptic Soy Agar (Difco) seeded with Micrococcus luteus. Plates were incubated overnight at 37^{o}C. An halo corresponding to an antimicrobial activity was detected when the complete reaction was carried out with extract corresponding to pIM-Cic transformed cells but was absent when the extracts were from cells transformed with pIM-IIIA1, when the substrate was obmitted or when the extract was substituted by lysis buffer. This antimicrobial activity most probably corresponds to isopenicillin N. In fact, antimicrobial activity is destroyed by penicillinase (80 units/ml), in agreement with this assumption. This example confirms that active isopenicillin N synthetase is produced in E. coli transformed with pIM-Cic.

Appendix 1
Nucleotide sequence of the DNA compound object of the present invention
10 20 30 40 50 60
ATGGGTTCAG TCAGCAAAGC CAATGTTCCA AAGATCGATG TTTCTCCCCT GTTTGGAGAC
TACCCAAGTC AGTCGTTTCG GTTACAAGGT TTCTAGCTAC AAAGAGGGGA CAAACCTCTG

70 80 90 100 110 120
GACCAAGCAG CCAAAATGAG AGTAGCCCAG CAAATCGACG CCGCTTCGCG TGATACTGGG
CTGGTTCGTC GGTTTTACTC TCATCGGGTC GTTTAGCTGC GGCGAAGCGC ACTATGACCC

130 140 150 160 170 180
TTTTTCTACG CCGTCAACCA TGGGATCAAT GTGCAGCGAC TCTCGCAGAA GACCAAGGAG
AAAAAGATGC GGCAGTTGGT ACCCTAGTTA CACGTCGCTG AGAGCGTCTT CTGGTTCCTC

190 200 210 220 230 240
TTTCATATGT CTATCACACC TGAGGAAAAG TGGGACCTTG CGATTCGTGC CTACAACAAA
AAAGTATACA GATAGTGTGG ACTCCTTTTC ACCCTGGAAC GCTAAGCACG GATGTTGTTT

250 260 270 280 290 300
GAGCACCAGG ACCAAGTCCG TGCCGGATAC TACCTGTCCA TCCCCGGGAA AAAGGCAGTC
CTCGTGGTCC TGGTTCAGGC ACGGCCTATG ATGGACAGGT AGGGGCCCTT TTTCCGTCAG

310 320 330 340 350 360
GAGTCCTTCT GCTACCTTAA CCCGAACTTC ACGCCCGATC ATCCCCGTAT CCAGGCCAAG
CTCAGGAAGA CGATGGAATT GGGCTTGAAG TGCGGGCTAG TAGGGGCATA GGTCCGGTTC

370 380 390 400 410 420
ACTCCCACTC ACGAGGTAAA CGTGTGGCCA GACGAGACCA AGCACCCTGG TTTCCAAGAC
TGAGGGTGAG TGCTCCATTT GCACACCGGT CTGCTCTGGT TCGTGGGACC AAAGGTTCTG

430 440 450 460 470 480
TTTGCCGAGC AGTATTACTG GGATGTTTTC GGTCTCTCTT CTGCACTGCT CAAGGGCTAC
AAACGGCTCG TCATAATGAC CCTACAAAAG CCAGAGAGAA GACGTGACGA GTTCCCGATG

490 500 510 520 530 540
GCCTTGGCAT TAGGCAAAGA GGAGAATTTC TTCGCTCGCC ACTTCAAGCC AGACGATACT
CGGAACCGTA ATCCGTTTCT CCTCTTAAAG AAGCGAGCGG TGAAGTTCGG TCTGCTATGA

550 560 570 580 590 600
CTTGCCTCGG TTGTGCTGAT CCGCTACCCT TACCTGGATC CCTACCCCGA GGCTGCTATC
GAACGGAGCC AACACGACTA GGCGATGGGA ATGGACCTAG GGATGGGGCT CCGACGATAG

610 620 630 640 650 660
AAGACGGCGG CCGACGGCAC CAAACTGAGT TTTGAGTGGC ATGAGGATGT ATCCCTAATC
TTCTGCCGCC GGCTGCCGTG GTTTGACTCA AAACTCACCG TACTCCTACA TAGGGATTAG

670 680 690 700 710 720
ACTGTGCTTT ACCAGTCCAA CGTGCAGAAC CTGCAGGTAG AAACTGCTGC CGGGTATCAA
TGACACGAAA TGGTCAGGTT GCACGTCTTG GACGTCCATC TTTGACGACG GCCCATAGTT

730 740 750 760 770 780
GATATCGAGG CCGACGATAC TGGCTACTTG ATCAACTGCG GCAGTTACAT GGCACATCTC
CTATAGCTCC GGCTGCTATG ACCGATGAAC TAGTTGACGC CGTCAATGTA CCGTGTAGAG

790 800 810 820 830 840
ACAAACAACT ACTATAAAGC GCCCATCCAT CGGGTGAAAT GGGTTAATGC AGAGCGCCAG
TGTTTGTTGA TGATATTTCG CGGGTAGGTA GCCCACTTTA CCCAATTACG TCTCGCGGTC

850 860 870 880 890 900
TCCCTGCCAT TCTTCGTCAA CCTGGGATAC GACAGCGTGA TTGATCCATT TGATCCCCGA
AGGGACGGTA AGAAGCAGTT GGACCCTATG CTGTCGCACT AACTAGGTAA ACTAGGGGCT

910 920 930 940 950 960
GAACCCAATG GCAAGTCTGA TCGGGAGCCA CTCTCCTACG GCGACTATTT GCAAAACGGG
CTTGGGTTAC CGTTCAGACT AGCCCTCGGT GAGAGGATGC CGCTGATAAA CGTTTTGCCC

970 980 990 3 13 23
CTGGTGAGTT TGATCAACAA GAACGGCCAG ACCTAG
GACCACTCAA ACTAGTTGTT CTTGCCGGTC TGGATC

Appendix 2.

Amino acid sequence of the polypeptide encoded in the novel DNA compound
object of the present invention. ATG encodes for methionine. Amino acids
are shown in the standard three letter code.

ATG-Gly-Ser-Val-Ser-Lys-Ala-Asn-Val-Pro-Lys-Ile-Asp-Val-Ser-Pro-Leu-Phe-Gly-Asp-
Asp-Gln-Ala-Ala-Lys-Met-Arg-Val-Ala-Gln-Gln-Ile-Asp-Ala-Ala-Ser-Arg-Asp-Thr-Gly-
Phe-Phe-Tyr-Ala-Val-Asn-His-Gly-Ile-Asn-Val-Gln-Arg-Leu-Ser-Gln-Lys-Thr-Lys-Glu-
Phe-His-Met-Ser-Ile-Thr-Pro-Glu-Glu-Lys-Trp-Asp-Leu-Ala-Ile-Arg-Ala-Tyr-Asn-Lys-
Glu-His-Gln-Asp-Gln-Val-Arg-Ala-Gly-Tyr-Tyr-Leu-Ser-Ile-Pro-Gly-Lys-Lys-Ala-Val-
Glu-Ser-Phe-Cys-Tyr-Leu-Asn-Pro-Asn-Phe-Thr-Pro-Asp-His-Pro-Arg-Ile-Gln-Ala-Lys-
Thr-Pro-Thr-His-Glu-Val-Asn-Val-Trp-Pro-Asp-Glu-Thr-Lys-His-Pro-Gly-Phe-Gln-Asp-
Phe-Ala-Glu-Gln-Tyr-Tyr-Trp-Asp-Val-Phe-Gly-Leu-Ser-Ser-Ala-Leu-Leu-Lys-Gly-Tyr-
Ala-Leu-Ala-Leu-Gly-Lys-Glu-Glu-Asn-Phe-Phe-Ala-Arg-His-Phe-Lys-Pro-Asp-Asp-Thr-
Leu-Ala-Ser-Val-Val-Leu-Ile-Arg-Tyr-Pro-Tyr-Leu-Asp-Pro-Tyr-Pro-Glu-Ala-Ala-Ile-
Lys-Thr-Ala-Ala-Asp-Gly-Thr-Lys-Leu-Ser-Phe-Glu-Trp-His-Glu-Asp-Val-Ser-Leu-Ile-
Thr-Val-Leu-Tyr-Gln-Ser-Asn-Val-Gln-Asn-Leu-Gln-Val-Glu-Thr-Ala-Ala-Gly-Tyr-Gln-
Asp-Ile-Glu-Ala-Asp-Asp-Thr-Gly-Tyr-Leu-Ile-Asn-Cys-Gly-Ser-Tyr-Met-Ala-His-Leu-
Thr-Asn-Asn-Tyr-Tyr-Lys-Ala-Pro-Ile-His-Arg-Val-Lys-Trp-Val-Asn-Ala-Glu-Arg-Gln-
Ser-Leu-Pro-Phe-Phe-Val-Asn-Leu-Gly-Tyr-Asp-Ser-Val-Ile-Asp-Pro-Phe-Asp-Pro-Arg-
Glu-Pro-Asn-Gly-Lys-Ser-Asp-Arg-Glu-Pro-Leu-Ser-Tyr-Gly-Asp-Tyr-Leu-Gln-Asn-Gly-
Leu-Val-Ser-Leu-Ile-Asn-Lys-Asn-Gly-Gln-Thr-Stop-

## Claims

1. A DNA sequence substantially encoding the Aspergillus nidulans isopenicillin N synthetase function having the amino acid sequence of Appendix 2.

2. A DNA sequence according to claim 1 wherein the A. nidulans IPNS function is encoded in the relevant portion of the plasmid pCiAnH2 contained in the E. coli strain deposited under NCIB 12450.

3. A DNA sequence according to claim 1 or claim 2 containing a coding sequence corresponding to that of Annex 1, or the degenerate equivalent thereof.

4. A sequence according to any preceding claim substantially encoding A. nidulans IPNS.

5. A vector comprising a sequence according to any preceding claim.

6. A vector according to claim 5 comprising the plasmid designated pCiAnH2 contained in the E. coli strain deposited under NCIB 12450.

7. A vector according to Claim 5 designated pIM-Cic which is constructed according to the following procedure:
(a) digestion of the plasmid pCiAnH2 contained in the E. coli strain deposited under NCIB 12450 with ClaI and BglII and isolation of a 1.7Kb fragment corresponding to the A. nidulans IPNS coding region;
(b) synthesis of an adaptor fragment having a sequence corresponding to the sequence between the XbaI site of the plasmid pIM-IIIA1 and the ATG corresponding to the lipoprotein coding region followed by the sequence between the initiation codon of the IPNS coding region and the cla1 site thereof, the adaptor having overhanging cla1 and Xba1 cohesive ends;
(c) digestion of the plasmid pIM-IIIA1 with XbaI, BamHI, HindIII and EcoRI;
(d) ligation of the fragments produced in (a), the plasmid fragments produced in (c) and the adaptor fragment produced in (b).

8. A vector derivable from the plasmid pCiAnH2 contained in the E. coli strain deposited under NCIB 12450 according to Claim 6, further comprising at least one A. nidulans transformation marker.

9. A vector derivable from the vector pIM-Cic according to Claim 7, further comprising at least one extra E. coli translational and/or transcriptional control signal.

10. A host cell containing at least one sequence according to any preceding claim in integrated or extra-chromosomal form.

11. A host cell according to claim 10 in which the host cell containing said sequence is A. nidulans, E. coli, Penicillium chrysogenum or Cephalosporium acremonium.

12. A host cell according to claim 10 in which the host cell containing said sequence is E. coli W3110.

13. A method for the production of an IPNS enzyme/proenzyme/preproenzyme comprising culturing a cell or cells according to any of claims 10 to 12 and isolating the derived product.

## Patentansprüche

1. DNA-Sequenz, welche im wesentlichen für die Funktion der Isopenicillin-N- Synthetase aus Aspergillus nidulans codiert und die Aminosäuresequenz des Anhangs 2 besitzt.

2. DNA-Sequenz nach Anspruch 1, worin die Funktion der IPNS aus A. nidulans im relevanten Teil des Plasmids pCiAnH2, welches in dem unter NCIB 12450 hinterlegten E. coli-Stamm enthalten ist, codiert ist.

3. DNA-Sequenz nach Anspruch 1 oder Anspruch 2, enthaltend eine codierende Sequenz, welche derjenigen des Anhangs 1 entspricht, oder das degenerierte Äquivalent davon.

4. Sequenz nach einem der vorstehenden Ansprüche, welche im wesentlichen für die IPNS aus A. nidulans codiert.

5. Vektor, umfassend eine Sequenz nach jedem vorstehenden Anspruch.

6. Vektor nach Anspruch 5, umfassend das als pCiAnH2 bezeichnete Plasmid, welches in dem unter NCIB 12450 hinterlegten E. coli- Stamm enthalten ist.

7. Vektor nach Anspruch 5, welcher als pIM-Cic bezeichnet und gem. dem folgenden Verfahren konstruiert ist:
(a) Spaltung des Plasmids pCiAnH2, welches in dem unter NCIB 12450 hinterlegten E. coli-Stamm enthalten ist, mit ClaI und BglII und Isolierung eines 1,7 Kb-Fragments, welches der Region, die für IPNS aus A. nidulans codiert, entspricht;
(b) Synthese eines Adaptor-Fragments, welches eine Sequenz besitzt, die der Sequenz zwischen der XbaI-Stelle des Plasmids pIM-IIIA1 und dem ATG, welches zu der für Lipoprotein codierenden Region gehört, gefolgt von der Sequenz zwischen dem Initiationscodon der für IPNS codierenden Region und deren ClaI-Stelle, wobei der Adaptor überhängende ClaI- und XbaI-Kohäsivenden besitzt;
(c) Spaltung des Plasmids pIM-IIIA1 mit XbaI, BamHI, HindIII und EcoRI;
(d) Ligierung des in (a) hergestellten Fragments, des in (c) hergestellten Plasmid-Fragments und des in (b) hergestellten Adaptor-Fragments.

8. Vektor, herleitbar vom Plasmid pCiAnH2, welches in dem unter NCIB 12450 hinterlegten E. coli-Stamm nach Anspruch 6 enthalten ist, weiterhin umfassend mindestens einen A. nidulans-Transformationsmarker.

9. Vektor, herleitbar vom Vektor pIM-Cic nach Anspruch 7, weiterhin umfassend mindestens ein weiteres E. coli-Translations- und/oder Transcriptionskontrollsignal.

10. Wirtzelle, enthaltend mindestens eine Sequenz nach jedem vorstehenden Anspruch in integrierter oder extrachromosomaler Form.

11. Wirtszelle nach Anspruch 10, worin die diese Sequenz enthaltende Wirtszelle A. nidulans, E. coli, Penicillium chrysogenum oder Cephalosporium acremonium ist.

12. Wirtszelle nach Anspruch 10, worin die diese Sequenz enthaltende Wirtszelle E. coli W3110 ist.

13. Verfahren zur Herstellung eines IPNS-Enzyms/Proenzyms/Präproenzyms, umfassend das Kultivieren einer Zelle oder von Zellen gem. einem der Ansprüche 10 bis 12 und Isolieren des hergestellten Produkts.

## Revendications

1. Séquence d'ADN encodant substantiellement la fonction synthétase de l'isopénicilline N de Aspergillus nidulans, ayant la séquence d'acides aminés de l'Annexe 2.

2. Séquence d'ADN selon la revendication 1, dans laquelle la fonction IPNS de A. nidulans est encodée dans la partie pertinente du plasmide pCiAnH2 contenu dans la souche de E. coli déposée sous le No NCIB 12450.

3. Séquence d'ADN selon la revendication 1 ou la revendication 2 contenant une séquence codante correspondant à celle de l'Annexe 1 ou son équivalent de dégénérescence.

4. Séquence selon l'une quelconque des revendications précédentes encodant substantiellement l'IPNS de A. nidulans.

5. Vecteur comprenant une séquence selon l'une quelconque des revendications précédentes.

6. Vecteur selon la revendication 5 comprenant le plasmide désigné par pCiAnH2 contenu dans la souche de E. coli déposée sous le No NCIB 12450.

7. Vecteur selon la revendication 5 désigné par pIM-Cic qui est construit suivant le mode opératoire suivant:
(a) digestion du plasmide pCiAnH2 contenu dans la souche de E. coli déposée sous le No NCIB 12450 avec ClaI et BglII et isolement d'un fragment de 1.7 Kb correspondant à la région codant pour IPNS de A. nidulans;
(b) synthèse d'un fragment adaptateur ayant une séquence correspondante à la séquence entre le site XbaI du plasmide pIM-IIIA1 et l'ATG correspondant à la région codant pour la lipoprotéine suivi par la séquence entre le codon d'initiation de la région codant pour IPNS et son site cla1, l'adaptateur ayant les terminaisons cohésives cla1 et Xba1 surplombantes;
(c) digestion du plasmide pIM-IIIA1 avec XbaI, BamHI, HindIII et EcoRI;
(d) ligation des fragments produits dans (a), des fragments de plasmide produits dans (c) et du fragment adaptateur produit dans (b).

8. Vecteur pouvant être dérivé du plasmide pCiAnH2 contenu dans la souche de E. coli déposée sous le No NCIB 12450 selon la revendication 6, comprenant en outre au moins un marqueur de transformation de A. nidulans.

9. Vecteur pouvant être dérivé du vecteur pIM-Cic selon la revendication 7, comprenant en outre un signal de contrôle transcriptionnel et/ou translationnel de E. coli supplémentaire.

10. Cellule hôte contenant au moins une séquence selon l'une quelconque des revendications précédentes dans une forme intégrée ou extrachromosomique.

11. Cellule hôte selon la revendication 10, dans laquelle la cellule hôte contenant ladite séquence est A. nidulans, E. coli, Penicillium chrysogenum ou Cephalosporium acremonium.

12. Cellule hôte selon la revendication 10, dans laquelle la cellule hôte contenant ladite séquence est E. coli W3110.

13. Procédé pour la production d'une enzyme/proenzyme/préproenzyme de IPNS comprenant la culture d'une cellule ou de cellules suivant l'une quelconque des revendications 10 à 12 et l'isolement du produit dérivé.
